(19) Europäisches Patentamt
European Patent Office
Office européen des brevets

(11) **EP 4 699 623 A1**

(12) **EUROPEAN PATENT APPLICATION**
published in accordance with Art. 153(4) EPC

(43) Date of publication:
**25.02.2026 Bulletin 2026/09**

(21) Application number: **24793077.9**

(22) Date of filing: **19.04.2024**

(51) International Patent Classification (IPC):
***A61L 27/20*** *(2006.01)* ***A61L 27/36*** *(2006.01)*
***A61L 27/26*** *(2006.01)* ***A61L 27/58*** *(2006.01)*

(52) Cooperative Patent Classification (CPC):
**A61L 27/20; A61L 27/26; A61L 27/36; A61L 27/58**

(86) International application number:
**PCT/KR2024/005349**

(87) International publication number:
**WO 2024/219893 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.04.2023 KR 20230051322**
**20.12.2023 KR 20230187031**

(71) Applicant: **PharmaResearch Co., Ltd.**
**Gangneung-si, Gangwon-do 25452 (KR)**

(72) Inventors:
- **KIM, Hongtaek**
  **Seongnam-si, Gyeonggi-do 13453 (KR)**
- **OHK, Seulong**
  **Seongnam-si, Gyeonggi-do 13453 (KR)**
- **CHUN, Soyoung**
  **Seongnam-si, Gyeonggi-do 13453 (KR)**
- **JUNG, Hayoung**
  **Seongnam-si, Gyeonggi-do 13453 (KR)**

(74) Representative: **Cabinet Beau de Loménie**
**103, rue de Grenelle / CS 90800**
**75340 Paris Cedex 07 (FR)**

# (54) TISSUE RESTORATION COMPOSITION

(57) The present invention relates to a composition for tissue repair, comprising: a nucleic acid; and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan.

[FIG. 1]

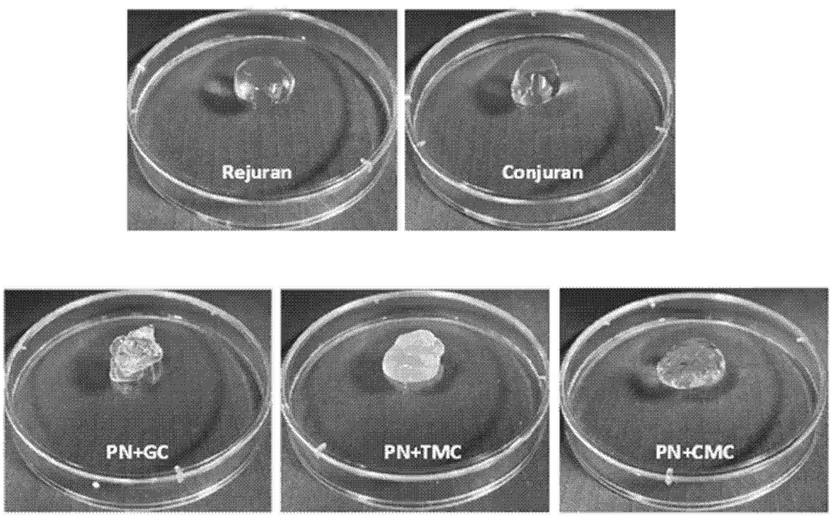




EP 4 699 623 A1

## Description

### [Technical Field]

The present invention relates to a composition for tissue repair, comprising: a nucleic acid; and one or more substances selected from the group consisting of glycol chitosan (GC), chitosan oligosaccharide lactate (COL), trimethyl chitosan (TMC), methyl glycol chitosan (MGC), carboxymethyl chitosan (CMC), N-hydroxymethyl chitosan, N-hydroxypropyl chitosan, and N-hydroxypropyl ether chitosan.

### [Background Art]

As the social structure changes and the population increases, the number of patients with burns, bedsores, trauma, plastic surgery, intractable ulcers, diabetic skin necrosis, *etc.* is also increasing, and treatment methods for damaged skin are also developing accordingly. For example, if more than 60% of the body surface area is damaged by a burn, it is common to die from sepsis, but recently, with the development of artificial skin, moisture loss and infection can be prevented, thereby significantly reducing the mortality rate. Furthermore, as interest in beauty increases, fillers are being widely used for cosmetic purposes.

Hyaluronic acid filler, which is currently the most widely used filler, accounts for more than 90% of the global filler market. In addition, a product in which hyaluronic acid and crosslinking agents are crosslinked to extend the resorption period is commercially available (Korean Patent Publication No. 10-2014-0072008). However, such crosslinked products have also been reported to cause problems due to the toxicity of the crosslinking agent. Accordingly, there remains a need for the development of compositions for tissue repair that have no risk of toxicity and exhibit excellent physical properties of biocompatibility and safety.

### [Disclosure]

### [Technical Problem]

A problem to be solved by the present invention is to provide a composition for tissue repair, comprising: a nucleic acid; and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan.

### [Technical Solution]

An object of the present invention is to provide a composition for tissue repair, comprising: a nucleic acid; and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan.

### [Advantageous Effects]

The composition of the present invention does not require chemical crosslinking, thereby exhibiting excellent stability, does not require a separate sterilization process, and exhibits functionality, physical properties, and safety suitable for use as a biomaterial for tissue repair. Thus, the composition can be usefully employed as a composition for various tissue repairs.

### [Brief Description of the Drawings]

FIG. 1 shows the physical properties of compositions for tissue repair of the present invention (Experimental Examples) and existing products, Rejuran and Conjuran (Comparative Examples).

FIG. 2 is a schematic diagram of an experimental plan for evaluating the tissue repair ability and biodegradability of a tissue repair composition.

FIG. 3 shows a volume measurement image using a three-dimensional imaging system (PRIMOSCR).

FIG. 4 shows a graph of the results of volume change analysis of the untreated group (saline) using a three-dimensional imaging system (PRIMOSCR).

FIG. 5 shows a graph of the results of volume change analysis of Test Group 1 (CJR) using a three-dimensional imaging system (PRIMOSCR).

FIG. 6 shows a graph of the results of volume change analysis of Test Group 2 (RJR) using a three-dimensional imaging system (PRIMOSCR).

FIG. 7 shows a graph of the results of volume change analysis of Test Group 3 (GC) using a three-dimensional imaging system (PRIMOSCR).

FIG. 8 shows a graph of the results of volume change analysis of Test Group 4 (TMC) using a three-dimensional imaging system (PRIMOSCR).

FIG. 9 shows a graph of the results of volume change analysis of Test Group 5 (CMC) using a three-dimensional imaging system (PRIMOSCR).

FIG. 10 shows an image of volume change analysis in mice using a three-dimensional imaging system (PRIMOSCR).

FIG. 11 shows a graph of the results of group-wise half-life analysis using a three-dimensional imaging system (PRIMOSCR).

## [Detailed Description of Preferred Embodiments]

The present invention will be described in detail as follows. Meanwhile, each description and embodiment described herein may be applied to other descriptions and embodiments, respectively. That is, all combinations of various elements described herein fall within the scope of the present invention. Further, the scope of the present invention is not limited by the specific description described below.

Furthermore, a person skilled in the art may recognize or identify numerous equivalents to the specific embodiments of the present invention described herein by using routine experimentation. Moreover, such equivalents are intended to be included within the present invention.

An aspect of the present invention provides a composition for tissue repair, comprising: a nucleic acid; and one or more substances selected from the group consisting of glycol chitosan (GC), chitosan oligosaccharide lactate (COL), trimethyl chitosan (TMC), methyl glycol chitosan (MGC), carboxymethyl chitosan (CMC), N-hydroxymethyl chitosan, N-hydroxypropyl chitosan, and N-hydroxypropyl ether chitosan.

In an embodiment according to any one of the preceding embodiments, the nucleic acid included in the composition of the present invention may be included in an amount of 0.1 wt% to 5 wt% based on the total weight of the composition.

In an embodiment according to any one of the preceding embodiments, any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be included in an amount of 0.01 wt% to 2 wt% based on the total weight of the composition.

In an embodiment according to any one of the preceding embodiments, the weight ratio of the nucleic acid to any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be from 500:1 to 1:20.

The nucleic acid of the present invention may be deoxyribonucleic acid (DNA), ribonucleic acid (RNA), or a mixture thereof. Specifically, the nucleic acid may be DNA, but is not limited thereto.

In an embodiment according to any one of the preceding embodiments, the nucleic acid of the present invention may be selected from an oligonucleotide, a polynucleotide, and a polydeoxyribonucleotide.

As used herein, the term "polynucleotide", also referred to as "PN", may refer to a DNA or RNA strand, which is a polymer of nucleotides in which nucleotide monomers are connected in a chain shape by covalent bonds. As used herein, the term "polydeoxyribonucleotide", also referred to as "PDRN", may be a type of low molecular weight DNA complex having a specific standard molecular weight, but is not limited thereto.

For example, the polynucleotide may have a relatively longer nucleic acid length or greater molecular weight than the polydeoxyribonucleotide and may be used as a raw material for a medical device by serving as a physical support for cell adhesion and providing lubrication and buffering effects, and the polydeoxyribonucleotide may be employed as a raw material for a pharmaceutical product for cell proliferation and tissue regeneration. However, the polynucleotide and the polydeoxyribonucleotide are not limited thereto.

In an embodiment according to any one of the preceding embodiments, the nucleic acid of the present invention may have a molecular weight of about 1 kDa to 100,000 kDa, 5 kDa to 50,000 kDa, 50 kDa to 10,000 kDa, or 50 kDa to 1,500 kDa.

In an embodiment according to any one of the preceding embodiments, the nucleic acid of the present invention may be obtained by extraction from the testis or semen of a fish. Specifically, the fish may be a Salmonidae fish. More specifically, the fish may be a salmon or a trout, but is not limited thereto.

As used herein, "glycol chitosan, chitosan oligosaccharide lactate, trimethyl chitosan, methyl glycol chitosan, carboxymethyl chitosan, N-hydroxymethyl chitosan, N-hydroxypropyl chitosan, or N-hydroxypropyl ether chitosan", which consist of components of the composition for tissue repair together with a nucleic acid, are substances that may be mixed with a nucleic acid to be provided as a composition for tissue repair.

Specifically, the substance of the present invention may be selected from glycol chitosan (GC), trimethyl chitosan (TMC), or carboxymethyl chitosan (CMC), but is not limited thereto.

As used herein, "glycol chitosan (GC)" may be a compound represented by Formula 1 below:

[Formula 1]

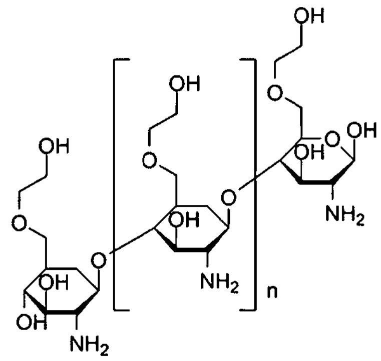

As used herein, "trimethyl chitosan (TMC)" is a multi-functional polymer that can be used in various nanoparticle forms in the pharmaceutical, functional food, and biopharmaceutical fields. The trimethyl chitosan may be a compound represented by Formula 2 below:

[Formula 2]

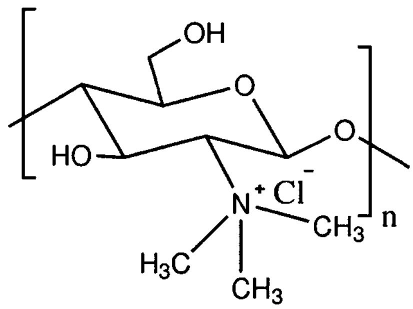

As used herein, "carboxymethyl chitosan (CMC)" refers to a compound formed by binding a carboxymethyl group to the C-2, C-6 position of chitosan, and it is characterized by properties such as biodegradability, biocompatibility, and antimicrobial properties, and is water soluble. The carboxymethyl chitosan may be a compound represented by Formula 3 below:

[Formula 3]

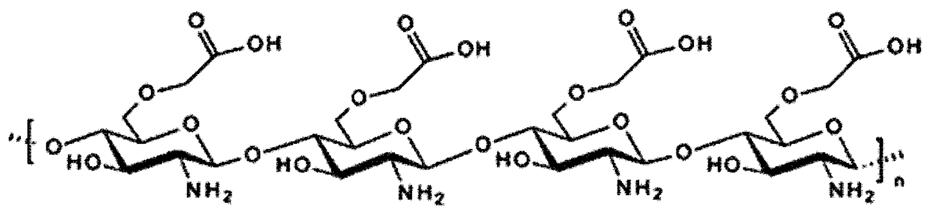

In an embodiment according to any one of the preceding embodiments, the substance of the present invention, that is, glycol chitosan, chitosan oligosaccharide lactate, trimethyl chitosan, methyl glycol chitosan, carboxymethyl chitosan, N-hydroxymethyl chitosan, N-hydroxypropyl chitosan, or N-hydroxypropyl ether chitosan, may be water-soluble.

In an embodiment according to any one of the preceding embodiments, any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan of the present invention may be dissolved in a neutral solvent having a pH of 5.0 to 9.0, and specifically, may be dissolved in a neutral solvent having a pH of 6.0 to 8.0.

The composition of the present invention may be suitable for use as a composition for tissue repair by including a nucleic acid and any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan in specific amounts.

In an embodiment according to any one of the preceding embodiments, the nucleic acid included in the composition of the present invention may be included in an amount of 0.1 wt% to 5 wt% based on the total weight of the composition.

Specifically, the nucleic acid included in the composition of the present invention may be about 0.3 wt% to 5 wt%, 0.5 wt% to 4 wt%, 0.7 wt% to 3 wt%, 1 wt% to 2.8 wt%, 1.2 wt% to 2.7 wt%, or 1.5 wt% to 2.5 wt% based on the total weight of the composition, but the content is not limited thereto.

In an embodiment according to any one of the preceding embodiments, any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be included in an amount of 0.01 wt% to 2 wt% based on the total weight of the composition.

Specifically, any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be included in an amount of about 0.01 wt% to 1.8 wt%, 0.05 wt% to 1.6 wt%, 0.07 wt% to 1.4 wt%, 0.08 wt% to 1.2 wt%, 0.09 wt% to 1.0 wt%, or 0.1 wt% to 0.5 wt% based on the total weight of the composition, but the content is not limited thereto.

In an embodiment according to any one of the preceding embodiments, the weight ratio of the nucleic acid to one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be 500:1 to 1:20.

Specifically, the weight ratio of the nucleic acid to one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan included in the composition of the present invention may be about 500:1, 400:1, 300:1, 200:1, 150:1, 100:1, 50:1, 25:1, 20:1, 15:1, 10:1, 5:1, 4:1, 3:1, 2:1, 5:4, 4:3, 3:2, 1:1, 2:3, 3:4, 4:5, 1:2, 1:3, 1:4, 1:5, 1:6, 1:7, 1:8, 1:9, 1:10, 1:12, 1:14, 1:16, 1:18, or 1:20, but is not limited thereto.

The term "about" encompasses not only the precise numerical value that is specified after the term but also a range that is approximately or nearly close to that value. Considering the context in which the number is presented, it can be determined whether the specific number mentioned is close to or nearly that number. In one example, the term "about" may refer to a range of -10% to +10% of the numerical value. In another example, the term "about" may refer to a range of -5% to +5% of the given numerical value. In still another example, the term "about" may refer to a range encompassing $\pm0.5$, $\pm0.4$, $\pm0.3$, $\pm0.2$, $\pm0.1$, *etc.,* but the range is not limited thereto.

The composition of the present invention may be dissolved in a neutral solvent without precipitation.

As used herein, "neutral" refers to a condition having a pH of about 5.0 to 9.0. Specifically, it may refer to a condition having a pH of about 5.5 to 8.5, and more specifically, it may refer to a condition having a pH of about 6.0 to 8.0, but is not limited thereto.

Any solvent may be used as the solvent of the present invention without limitation, as long as it can dissolve a nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan. For example, the solvent may be a polar solvent or a hydrophilic solvent, and a specific example may be water, but the solvent is not limited thereto.

In one embodiment, the composition of the present invention may be dissolved in a polar solvent having a pH of 5.0 to 9.0 without precipitation, and more specifically, may be dissolved in water having a pH of 6.0 to 8.0 without precipitation, but the solvent is not limited thereto.

In an embodiment according to any one of the preceding embodiments, water for injection may be used as the solvent of the composition of the present invention, and unless otherwise specified, physiological saline, Ringer's solution, or other suitable aqueous solvents may be used in place of water for injection, but the solvent is not limited thereto.

The composition of the present invention may further include a water-soluble polymer.

Specifically, the water-soluble polymer may be one or more selected from the group consisting of hyaluronic acid, chondroitin sulfate, glycogen, dextrin, dextran, dextran sulfate, hydroxypropyl methylcellulose, alginic acid, chitin, pullulan, collagen, gelatin and hydrolysates thereof, polyvinyl alcohol, polyvinyl pyrrolidone, polyacrylic acid, and carboxyvinyl polymer.

The water-soluble polymer of the present invention may be soluble or degradable *in vivo.* The water-soluble polymer may, for example, function to control the degradation rate of the composition for tissue repair of the present invention or to provide lubrication, but is not limited thereto.

The term "for tissue repair" may encompass all biomaterials used for the replacement, repair, or reconstruction of human tissues and organs such as blood vessels, heart, diaphragm, fascia, and skin (e.g., dermal, cutaneous), and may also be used in a broad sense to include products containing secondarily processed human-derived skin in which additives are mixed to enhance function. Additionally, it may be used interchangeably with the term "biomaterial for tissue repair".

Specifically, it may refer to a substance that is inserted into a specific area with a component similar to human tissue to expand soft tissue and fill in sunken or lost areas, thereby improving wrinkles, correcting contours, and enabling use as a synovial fluid supplement. It can mean temporarily or semi-permanently improving or repairing wrinkles on the skin of the body, improving contours, creating volume in tissues, or regenerating tissues such as scar healing. The skin and tissue represent the face, chest, buttocks, genitals, and other body parts.

The composition for tissue repair of the present invention may be used as a cosmetic filler, a plastic prosthesis, a joint repair composition, or a combination thereof, but is not limited thereto. It may be used without limitation as long as it can be applied as a biomaterial.

The composition of the present invention may have physical properties suitable for use in tissue repair. For example, the composition of the present invention may exhibit elasticity and/or viscosity that are similar or superior to those of existing products capable of being injected into a target tissue.

As used herein, the term "elasticity" refers to the property of a solid in which the shape changes upon application of force and returns to its original shape when the force is removed. It indicates the degree to which a desired shape is maintained under the skin tissue, and the higher the elasticity, the longer the initially treated shape is maintained. Such elasticity is expressed as the elastic modulus or the storage modulus (G'), and the unit is Pascal (Pa).

In one embodiment, the composition of the present invention may exhibit an elasticity of about 80 Pa to 250 Pa at a temperature of 25°C. For example, the composition exhibits an elastic modulus of about 80 Pa to about 250 Pa, about 90 Pa to about 225 Pa, about 100 Pa to about 200 Pa, about 110 Pa to about 190 Pa, about 120 Pa to about 180 Pa, or about 130 Pa to about 160 Pa. In another embodiment, the composition for tissue repair of the present invention exhibits an elastic modulus of about 80 Pa or more, about 90 Pa or more, about 100 Pa or more, about 110 Pa or more, about 120 Pa or more, about 130 Pa or more, about 140 Pa or more, about 150 Pa or more, about 160 Pa or more, about 170 Pa or more, about 180 Pa or more, about 190 Pa or more, about 200 Pa or more, about 225 Pa or more, or about 250 Pa or more.

When the range of elastic modulus is included in the above range, a uniform tissue repair effect may be achieved upon *in vivo* administration, and a higher elastic modulus may lead to a longer *in vivo* retention period.

"Viscosity" of the present invention is a quantity representing the magnitude of viscosity, which refers to the resistance of a fluid to flow. Higher viscosity allows for easier surgical handling and enables the formation of delicate shapes.

In one embodiment, the composition of the present invention may exhibit a viscosity of about 100 Pa·s to 400 Pa·s under the conditions of a temperature of 25°C and a shear rate of 0.1 $\cdot s^{-1}$. For example, the composition exhibits a viscosity of about 100 Pa·s to about 400 Pa·s, about 120 Pa·s to about 380 Pa·s, about 130 Pa·s to about 360 Pa·s, about 140 Pa·s to about 340 Pa·s, about 150 Pa·s to about 320 Pa·s, about 160 Pa·s to about 300 Pa·s, about 170 Pa·s to about 280 Pa·s, about 180 Pa·s to about 260 Pa·s, about 190 Pa·s to about 240 Pa·s, about 200 Pa·s to about 260 Pa·s, about 220 Pa·s to about 280 Pa·s, about 240 Pa·s to about 300 Pa·s, or about 260 Pa·s to about 320 Pa·s. In another embodiment, the composition for tissue repair of the present invention exhibits an elastic modulus of about 100 Pa·s or more, about 120 Pa·s or more, about 150 Pa·s or more, about 180 Pa·s or more, about 200 Pa·s or more, about 220 Pa·s or more, about 240 Pa·s or more, about 260 Pa·s or more, about 280 Pa·s or more, about 300 Pa·s or more, about 320 Pa·s or more, about 340 Pa·s or more, about 360 Pa·s or more, about 380 Pa·s or more, or about 400 Pa·s or more.

It is important to inject a composition for tissue repair suitable for a target site by controlling the ranges of viscosity and elastic modulus within the above-mentioned ranges.

In an embodiment according to any one of the preceding embodiments, the composition of the present invention may be characterized by volume retention.

In an embodiment according to any one of the preceding embodiments, the composition of the present invention may be characterized by biodegradability.

For use in tissue repair, the composition should have appropriate volume retention in addition to biocompatibility to form volume in human tissue. Additionally, it may be appropriate for the composition to also have biodegradability such that it decomposes after a certain period of time and can be naturally eliminated through the body's metabolic processes. Without biocompatibility or appropriate volume retention, there may be difficulties in improving or repairing wrinkles on the skin of the body, improving contours, forming volume in tissues, or regenerating tissues such as scar healing. Additionally, if it is not biodegradable in the body, it may cause harmful side effects such as allergic reactions in the body.

The composition of the present invention was confirmed to have no risk of toxicity and to exhibit excellent physical properties of biocompatibility, safety, and appropriate tissue repair ability and/or biodegradability.

For example, the results of evaluating the tissue repair ability and/or biodegradability of the composition of the present invention through animal testing confirmed that the composition exhibited tissue repair ability and biodegradability levels similar to those of comparative examples, Conjuran and Rejuran. Through the above, it was confirmed that the composition of the present invention has volume retention, can repair tissue for a certain period of time, and has appropriate biodegradability, so that it can be reliably used as a biomaterial for tissue repair.

Another aspect of the present invention provides a preparation method for a composition for tissue repair, comprising dissolving, in a solvent, a nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan.

The solvent used in the preparation method of the present invention is not particularly limited as long as the solvent can dissolve the nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan. Specifically, a neutral solvent may be used. Specifically, the solvent may be water, but is not limited thereto.

The solvent of the present invention does not require the addition of an additional acidic solution to dissolve the mixture due to the characteristic of a mixture of a nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan. Moreover, it is not necessary to add any raw materials or solutions to neutralize effects that may result from the composition itself or from its administration into the body, thereby making it suitable for use as an injectable formulation.

In the dissolving step, the order of mixing a nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan with a solvent may be in any order, and dissolving in a simultaneous manner is also possible.

The preparation method for the composition for tissue repair of the present invention may further comprise treatment including degassing, disinfection, and sterilization.

In one embodiment, the preparation method of the present invention may further comprise filtering before and/or after the dissolving step. In particular, the type of filter used may be changed appropriately according to the purpose, and for example, a 0.20 μm to 0.50 μm filter may be used, but the type of filter is not limited thereto.

In one embodiment, the preparation method of the present invention may comprise a sterilization process.

The sterilization process may be performed using any known method without limitation. The sterilization process may include high-pressure sterilization, high-temperature sterilization, filtration, chemical treatment, and/or radiation treatment, but is not limited thereto.

The sterilization process of the present invention may be performed after the dissolving step, but is not limited thereto. For example, the sterilization process after the dissolving step may be high-temperature sterilization, but is not limited thereto.

In one embodiment according to any one of the preceding embodiments, the sterilization process may be performed at the final stage of the manufacturing process. The sterilization process performed at the final stage of the manufacturing process may be referred to as a "terminal sterilization process".

Specifically, the terminal sterilization process of the present invention may comprise a high-temperature sterilization process.

The preparation method of the present invention is characterized in that no precipitate is formed during sterilization after mixing the characteristic raw materials, a nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan. Accordingly, the preparation method of the present invention may not require a process of sterilizing each of the nucleic acid and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan before mixing, and mixing the sterilized raw materials in an aseptic facility.

A composition for tissue repair prepared according to the preparation method of the present invention may be filled into a container in a form suitable for use. For example, the composition may be provided in the form of a syringe, in which a single or multiple dose of the composition is filled in a vial within the syringe. In particular, the syringe may constitute a plunger, *etc.,* for extruding the drug, and thus, the composition of the present invention may be in an extrudable form..

Still another aspect of the present invention provides a method for tissue repair, comprising administering the composition for tissue repair of the present invention to a subject.

As used herein, the term "tissue repair" may be replacement, repair, or reconstruction of human tissues and

organs such as blood vessels, heart, diaphragm, fascia, and skin (e.g., dermal, cutaneous), and may be physical repair.

As used herein, the term "subject" may refer to any animal, including humans, in need of tissue repair. The animal may be a human or a mammal, but is not limited thereto.

As used herein, the term "administering" refers to introducing the composition for tissue repair of the present invention into a subject by a suitable method, and specifically by injection, but is not limited thereto. The route of administration of the composition of the present invention may include various routes, such as subcutaneous, dermal, blood vessel, biological membrane, tissue fiber, and synovial fluid, as long as tissue repair is possible. The dosage form may be a topical agent, subcutaneous injection, dermal injection, intravascular injection, intramuscular injection, intra-articular injection, tendon injection, or ligament injection, but is not limited thereto.

Yet another aspect of the present invention provides use of the composition for tissue repair of the present invention in tissue repair.

The terms "composition for tissue repair" and "tissue repair" are as described in other aspects.

**[Detailed Description of Preferred Embodiments]**

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

**Example 1: Preparation of Compositions for Tissue Repair Comprising a Nucleic Acid and GC, TMC, or CMC**

Compositions for tissue repair comprising a nucleic acid and glycol chitosan (GC), trimethyl chitosan (TMC), or carboxymethyl chitosan (CMC) were prepared. Conjuran (2 wt% PN, Pharma Research Co., Ltd.), used for joint space repair, and Rejuran (2 wt% PN, Pharma Research Co., Ltd.), used for facial repair, were used as comparative examples.

The components used in each composition and the content of each component based on the total composition are shown in Table 1 below.

[Table 1]

| Raw material | Weight% | | |
|---|---|---|---|
| | Test Group 3 | Test Group 4 | Test Group 5 |
| Nucleic acid | 2 | 2 | 2 |
| Glycol chitosan (GC) | 0.2 | 0 | 0 |
| Trimethyl chitosan (TMC) | 0 | 0.2 | 0 |
| Carboxymethyl chitosan (CMC) | 0 | 0 | 0.2 |
| - | - | - | - |
| NaCl | 0.8 | 0.8 | 0.8 |
| $Na_2HPO_412H_2O$ | 0.4 | 0.4 | 0.4 |
| Distilled water (D.W) | 96.6 | 96.6 | 96.6 |
| Pigs | 100 | 100 | 100 |

**Example 2: Evaluation of Physical Properties of Compositions for Tissue Repair**

The physical properties of Test Groups 3 to 5 prepared in Example 1 and those of the comparative examples, Test Groups 1 and 2, were compared and evaluated. The evaluation items and results are shown in Table 2 below.

[Table 2]

| Evaluation Item | Test Group 1 Conjuran (CJR) (2% PN) | Test Group 2 Rejuran (RJR) (2% PN) | Test Group 3 (2% PN + 0.2% GC) | Test Group 4 (2% PN + 0.2% TMC) | Test Group 5 (2% PN + 0.2% CMC) |
|---|---|---|---|---|---|
| **Elasticity (Pa, 25°C)** | 92.31 | - | 199.5 | 166.1 | 151.3 |

(continued)

| Evaluation Item | Test Group 1 Conjuran (CJR) (2% PN) | Test Group 2 Rejuran (RJR) (2% PN) | Test Group 3 (2% PN + 0.2% GC) | Test Group 4 (2% PN + 0.2% TMC) | Test Group 5 (2% PN + 0.2% CMC) |
|---|---|---|---|---|---|
| **Viscosity (Pa-s, 25°C, shear rate: 0.1 $s^{-1}$)** | 213.6 | 101.3 | 317.6 | 264.7 | 241.2 |
| **Appearance** | Colorless, trans-parent | Colorless, trans-parent | Colorless, transparent | Colorless, translucent | Colorless, transparent |
| "=" means unmeasured. | | | | | |

The experimental results showed that Test Groups 3 to 5, newly developed in the present invention exhibited properties similar to those of the comparative examples, Conjuran (Test Group 1) and Rejuran (Test Group 2), which are conventionally used as compositions for tissue repair, or even exhibited higher elasticity and viscosity. Thus, it was found that these compositions can be injected into a target tissue where volume retention or maintenance of post-procedural shape is required.

**Example 3: Evaluation of Tissue Repair Ability and Biodegradability of Compositions for Tissue Repair**

**Example 3-1: Evaluation of Tissue Repair Ability of Compositions for Tissue Repair**

In order to evaluate the tissue repair ability of the composition of the present invention, volume retention was measured through evaluation using a three-dimensional imaging system (PRIMOS$^{CR}$).

Specifically, the experimental animals, 18 male 6-week-old SKH-1 hairless mice, were purchased from Orient Bio Co., Ltd. and housed under controlled conditions (temperature: 20-24°C, humidity: 40-60%, differential pressure: negative pressure, noise: 60 dB or less, 12-hour light-dark cycle). The animals were acclimated for one week after arrival and then used for the experiment. Anesthesia was performed via inhalation using isoflurane maintained at a concentration of 3.0%. Once anesthesia was confirmed, the animals were transferred to the surgical table. Then, the untreated group (saline), Test Group 1 (CJR), Test Group 2 (RJR), Test Group 3 (GC), Test Group 4 (TMC), and Test Group 5 (CMC) were each subcutaneously administered 100 μL of the respective formulation into the back of the mice using a 1 mL syringe, with three mice per group. The administration time of the test substance is shown in Table 3 below, and the experimental schematic diagram is shown in FIG. 2.

[Table 3]

| Evaluation Item | 0 hr after adminis tration | 4 hr after adminis tration | 8 hr after adminis tration | 12 hr after adminis tration | 24 hr after adminis tration | 36 hr after adminis tration | 48 hr after adminis tration | 60 hr after adminis tration |
|---|---|---|---|---|---|---|---|---|
| Evaluation of three-dimensional imaging system (PRIMOS$^{CR}$) | ○ | ○ | ○ | ○ | ○ | ○ | ○ | O |

As shown in Table 3, the animals were anesthetized at 0, 4, 8, 12, 24, 36, 48, and 60 hours after the administration of the test substance by inhalation using isoflurane at a concentration of 3.0%. After fixing the camera, images were captured using a three-dimensional imaging system (PRIMOSCR, Canfield, USA) equipped with a high-resolution sensor, and volume measurement and analysis were performed using PRIMOS 5.0 software. Additionally, it was determined that the composition exhibits tissue repair ability when the volume was maintained at 70%.

All data produced were verified for statistical significance using SPSS Package Program version 20 (IBM, USA). The comparison of the evaluation results with the control group was performed using the ANOVA method (*p<0.05, **p<0.01, ***p<0.001).

$$\text{Volume change rate measured by 3D imaging system (PRIMOS}^{CR}\text{) (\%)} = \frac{\text{Volume at 4, 8, 12, 24, 36, 48, 60 hr (mm}^3\text{)}}{\text{Volume at 0 hr (mm}^3\text{)}} \times 100$$

The results of analyzing volume changes using the three-dimensional imaging system (PRIMOSCR) confirmed that the untreated group (saline) retained tissue repair ability for more than 4 hours and was degraded within 12 hours; Test Group 1 (CJR) retained tissue repair ability for more than 24 hours and was degraded within 48 hours; and Test Group 2 (RJR) retained tissue repair ability for more than 12 hours and was degraded within 48 hours. In addition, it was confirmed that Test Group 3 (GC) retained tissue repair ability for more than 24 hours and was degraded within 48 hours; Test Group 4 (TMC) retained tissue repair ability for more than 24 hours and was degraded within 48 hours; and Test Group 5 (CMC) retained tissue repair ability for 8 to 12 hours or more and was degraded within 48 hours (see Table 4 and FIGS. 4 to 10).

[Table 4]

| Evaluation of Three-Dimensional Imaging System (PRIMOS$^{CR}$) (N=3/group, total=18) | | | | | | | |
|---|---|---|---|---|---|---|---|
| Group | Test Substance | Time Point | Volume Analysis (mm$^3$) | | | [2]Significance Probability (*p*-value) (vs. 0 hr) | [3]Rate of Change (%) (vs. 0 hr) |
| | | | [1]Mean | SEM | SD | | |
| Untreated Group | Saline | 0 hr | 74.97 | 17.51 | 30.33 | - | - |
| | | 4 hr | 100.08 | 17.53 | 30.36 | 0.368 | 3349▲ |
| | | 8 hr | 18.25 | 2.42 | 4.19 | 0.033* | 7566▼ |
| | | 12 hr | 0.00 | 0.00 | 0.00 | 0.013* | 100.00▼ |
| | | 24 hr | 0.00 | 0.00 | 0.00 | 0.013* | 100.00▼ |
| | | 36 hr | 0.00 | 0.00 | 0.00 | 0.013* | 100.00▼ |
| | | 48 hr | 0.00 | 0.00 | 0.00 | 0.013* | 100.00▼ |
| | | 60 hr | 0.00 | 0.00 | 0.00 | 0.013* | 100.00▼ |
| Test Group 1 | CJR | 0 hr | 89.39 | 4.42 | 7.66 | - | - |
| | | 4 hr | 93.18 | 4.28 | 7.42 | 0.571 | 4.24 ▲ |
| | | 8 hr | 98.83 | 9.50 | 16.46 | 0.419 | 10.56 ▲ |
| | | 12 hr | 87.09 | 8.03 | 13.90 | 0.815 | 2.56▼ |
| | | 24 hr | 68.57 | 9.03 | 15.64 | 0.107 | 23.29▼ |
| | | 36 hr | 17.84 | 5.07 | 8.78 | 0.000*** | 80.04▼ |
| | | 48 hr | 0.00 | 0.00 | 0.00 | 0.000*** | 100.00▼ |
| | | 60 hr | 0.00 | 0.00 | 0.00 | 0.000*** | 100.00▼ |
| Test Group 2 | RJR | 0 hr | 118.27 | 5.01 | 8.68 | - | - |
| | | 4 hr | 137.79 | 0.84 | 1.45 | 0.018* | 16.50▲ |
| | | 8 hr | 108.32 | 6.88 | 11.92 | 0.307 | 8.41▼ |
| | | 12 hr | 81.41 | 7.66 | 13.26 | 0.016* | 31.17▼ |
| | | 24 hr | 45.41 | 6.28 | 10.87 | 0.001** | 61.61▼ |
| | | 36 hr | 20.20 | 2.28 | 3.95 | 0.000*** | 82.92▼ |
| | | 48 hr | 0.00 | 0.00 | 0.00 | 0.000*** | 100.00▼ |
| | | 60 hr | 0.00 | 0.00 | 0.00 | 0.000*** | 100.00▼ |

(continued)

| Evaluation of Three-Dimensional Imaging System (PRIMOS[CR]) (N=3/group, total=18) | | | | | | | |
|---|---|---|---|---|---|---|---|
| Group | Test Substance | Time Point | Volume Analysis (mm$^3$) | | | [2]Significance Probability (*p*-value) (vs. 0 hr) | [3]Rate of Change (%) (vs. 0 hr) |
| | | | [1]Mean | SEM | SD | | |
| Test Group 3 | GC | 0 hr | 124.64 | 5.39 | 9.34 | - | - |
| | | 4 hr | 129.17 | 3.63 | 6.29 | 0.524 | 3.63▼ |
| | | 8 hr | 108.16 | 8.75 | 15.16 | 0.184 | 13.22▼ |
| | | 12 hr | 93.16 | 4.17 | 7.22 | 0.010* | 2526▼ |
| | | 24 hr | 62.53 | 7.98 | 13.82 | 0.003** | 49.83▼ |
| | | 36 hr | 19.77 | 7.57 | 13.11 | 0.000*** | 8414▼ |
| | | 48 hr | 0.00 | 0.00 | 0.00 | 0.000*** | 100.00▼ |
| | | 60 hr | 0.00 | 0.00 | 0.00 | 0.000*** | 100.00▼ |
| Test Group 4 | TMC | 0 hr | 107.40 | 6.08 | 10.54 | - | - |
| | | 4 hr | 115.72 | 4.76 | 8.25 | 0.342 | 7.74▲ |
| | | 8 hr | 105.10 | 3.60 | 6.24 | 0.760 | 2.15▼ |
| | | 12 hr | 89.75 | 2.14 | 3.70 | 0.052 | 16.44▼ |
| | | 24 hr | 73.34 | 2.27 | 3.93 | 0.006** | 31.72▼ |
| | | 36 hr | 30.11 | 4.61 | 7.98 | 0.001** | 71.97▼ |
| | | 48 hr | 8.07 | 1.27 | 2.19 | 0.000*** | 9248▼ |
| | | 60 hr | 0.00 | 0.00 | 0.00 | 0.000*** | 100.00▼ |
| Test Group 5 | CMC | 0 hr | 116.75 | 4.05 | 7.02 | - | - |
| | | 4 hr | 124.96 | 6.63 | 11.48 | 0.351 | 7.03 A |
| | | 8 hr | 99 .07 | 14.10 | 24.43 | 0.295 | 15.14▼ |
| | | 12 hr | 74.94 | 10.82 | 18.75 | 0.022* | 3581▼ |
| | | 24 hr | 47.24 | 3.90 | 6.75 | 0.000*** | 59.54▼ |
| | | 36 hr | 15.90 | 8.35 | 14.46 | 0.000*** | 86.38▼ |
| | | 48 hr | 0.00 | 0.00 | 0.00 | 0.000*** | 100.00▼ |
| | | 60 hr | 0.00 | 0.00 | 0.00 | 0.000*** | 100.00▼ |
| [1] A decrease in the mean value indicates a decrease in the volume.<br>[2] If $p$ <0.05, there is a statistically significant difference compared to 0 hour (*$p$ <0.05, **$p$ <0.01, ***$p$ <0.001)<br>[3] Change rate (%) compared to 0 hour. | | | | | | | |

**Example 3-2: Evaluation of Biodegradability of Compositions for Tissue Repair**

To evaluate the biodegradability of the composition for tissue repair of the present invention, a half-life analysis evaluation was conducted.

Specifically, the trend of change between the time of maximum volume and the time of minimum volume was represented as an exponential function based on measurements taken at 0, 4, 8, 12, 24, 48, and 60 hours after administration of the test substances of the present invention. The X (time) value at which the Y (volume) value reached half of the maximum volume was then determined and analyzed.

All data produced were verified for statistical significance using SPSS Package Program version 20 (IBM, USA). The comparison of the evaluation results with the control group was performed using the ANOVA method (*p<0.05, **P<0.01, ***p<0.001).

As a result of the half-life (hr) analysis, it was confirmed that the half-life was 5.75 hours for the untreated group (saline), 21.45 hours for Test Group 1 (CJR), 17.15 hours for Test Group 2 (RJR), 18.40 hours for Test Group 3 (GC), 20.59

hours for Test Group 4 (TMC), and 16.69 hours for Test Group 5 (CMC) (see Table 5 and FIG. 11).

[Table 5]

| Half-life (hr) Analysis Results | | (N=3/group, total=18) | | |
|---|---|---|---|---|
| Group | Test Substance | Half-life (hr) | | |
| | | [1]Mean | SEM | SD |
| Untreated Group | Saline | 5.75 | 0.35 | 0.60 |
| Test Group 1 | CJR | 21.45 | 1.89 | 3.27 |
| Test Group 1 | RJR | 17.15 | 0.82 | 1.42 |
| Test Group 3 | GC | 18.40 | 2.42 | 4.19 |
| Test Group 4 | TMC | 17.96 | 0.56 | 0.98 |
| Test Group 5 | CMC | 16.69 | 1.21 | 2.09 |
| [1]A decrease in the mean value indicates a decrease in the half-life (hr). | | | | |

In summary, it was confirmed that the test substances of the present invention-Test Group 1 (CJR), Test Group 3 (GC), and Test Group 4 (TMC)-retained tissue repair ability for nearly 24 hours, while Test Group 2 (RJR) and Test Group 5 (CMC) retained tissue repair ability for 8 to 12 hours or more, and all groups were degraded within 48 hours.

Based on the above results, it was found that Test Groups 3 to 5, newly developed in the present invention, also exhibited tissue repair ability and biodegradability similar to those of the comparative examples, Conjuran (Test Group 1) and Rejuran (Test Group 2), which are conventionally used as compositions for tissue repair.

Accordingly, the test substances of the present invention exhibit volume retention, enabling tissue repair over a certain period of time, and exhibit appropriate biodegradability, suggesting that these test substances can be stably used as biomaterials for tissue repair.

From the above description, a person skilled in the art to which the present invention pertains would understand that the present invention can be implemented in other specific forms without changing its technical concept or essential features. Therefore, the embodiments described above should be construed as being exemplified and not limiting the present invention. The scope of the present invention should be interpreted to include all modifications or modified forms derived from the meaning and scope of the claims as set forth below, as well as equivalent concepts, rather than from the detailed description above.

## Claims

1. A composition for tissue repair, comprising: a nucleic acid; and one or more substances selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan.

2. The composition according to claim 1, wherein the nucleic acid is included in an amount of 0.1 wt% to 5 wt% based on the total weight of the composition.

3. The composition according to claim 1, wherein the any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan is included in an amount of 0.01 wt% to 2 wt% based on the total weight of the composition.

4. The composition according to claim 1, wherein the weight ratio of the nucleic acid to the any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan is from 500:1 to 1:20.

5. The composition according to claim 1, wherein the nucleic acid has a molecular weight of 50 kDa to 10,000 kDa.

6. The composition according to claim 1, wherein the nucleic acid is isolated from testis or semen of a fish.

7. The composition according to claim 6, wherein the fish is of the Salmonidae family.

8. The composition according to claim 1, wherein the substance is one or more selected from glycol chitosan, trimethyl chitosan, and carboxymethyl chitosan.

9. The composition according to claim 1, wherein the any one substance selected from the group consisting of glycol chitosan, chitosan oligosaccharide lactate, trimethyl chitosan, methyl glycol chitosan, carboxymethyl chitosan, N-hydroxymethyl chitosan, N-hydroxypropyl chitosan, and N-hydroxypropyl ether chitosan dissolve in a neutral solvent having a pH of 5 to 9.

10. The composition according to claim 1, wherein the composition further comprises a water-soluble polymer.

11. The composition according to claim 10, wherein the water-soluble polymer is one or more selected from the group consisting of: hyaluronic acid, chondroitin sulfate, glycogen, dextrin, dextran, dextran sulfate, hydroxypropyl methylcellulose, alginic acid, chitin, pullulan, collagen, gelatin and hydrolysates thereof, polyvinyl alcohol, polyvinyl pyrrolidone, polyacrylic acid, and carboxyvinyl polymer.

12. The composition according to claim 1, wherein the elasticity of the composition is 90 Pa to 250 Pa.

13. The composition according to claim 1, wherein the viscosity of the composition is 100 Pa·s to 400 Pa·s.

14. The composition according to claim 1, wherein the composition is **characterized by** volume retention.

15. The composition according to claim 1, wherein the composition is **characterized by** biodegradability.

[FIG. 1]

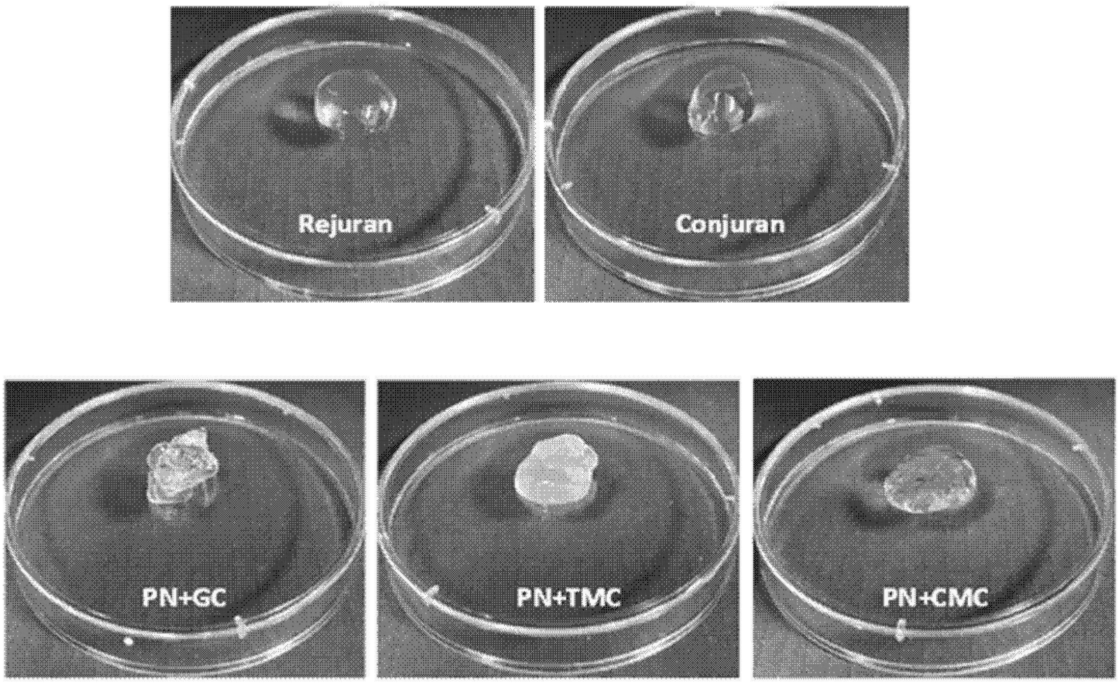
Rejuran
Conjuran
PN+GC
PN+TMC
PN+CMC

[FIG. 2]

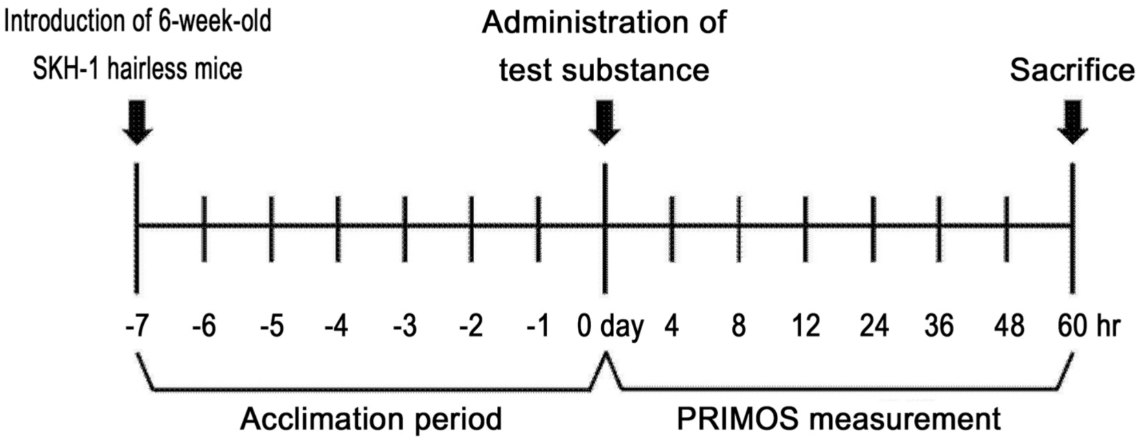
Introduction of 6-week-old SKH-1 hairless mice
Administration of test substance
Sacrifice
-7 -6 -5 -4 -3 -2 -1 0 day 4 8 12 24 36 48 60 hr
Acclimation period
PRIMOS measurement

[FIG. 3]

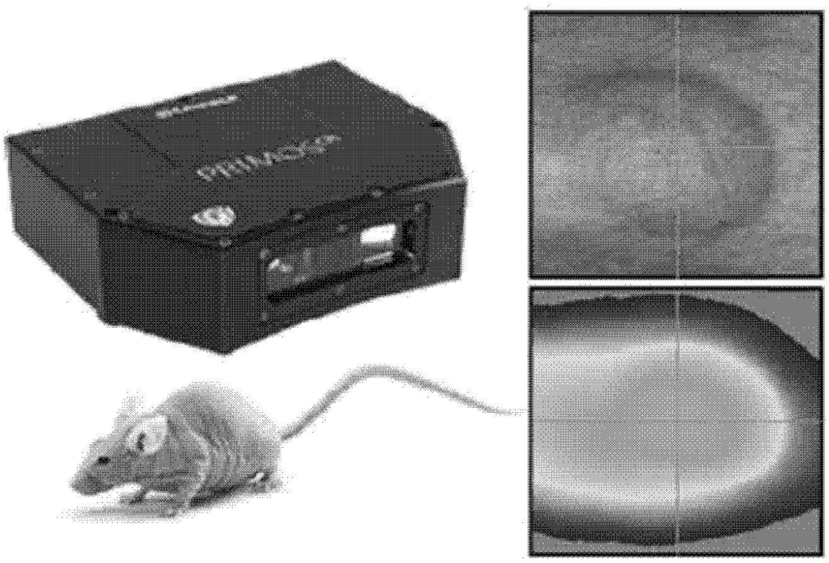
PRIMOS

[FIG. 4]

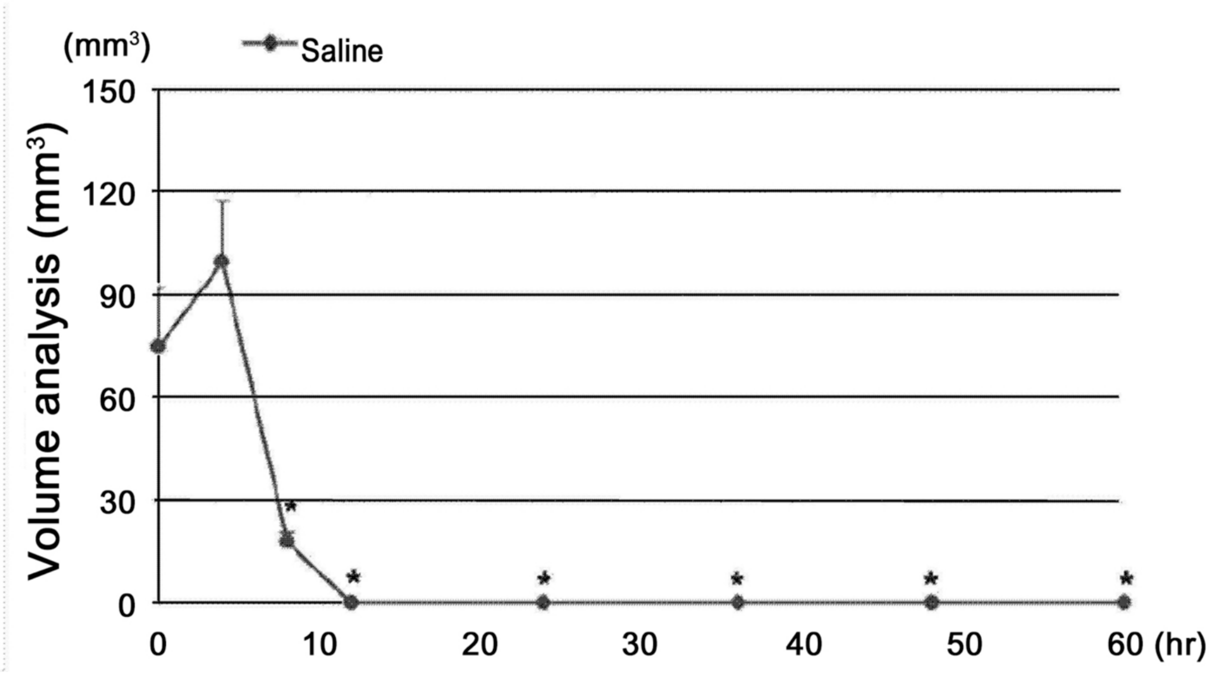
(mm3)
Saline
150
120
90
60
30
0
Volume analysis (mm3)
*
*
*
*
*
*
0
10
20
30
40
50
60 (hr)

[FIG. 5]

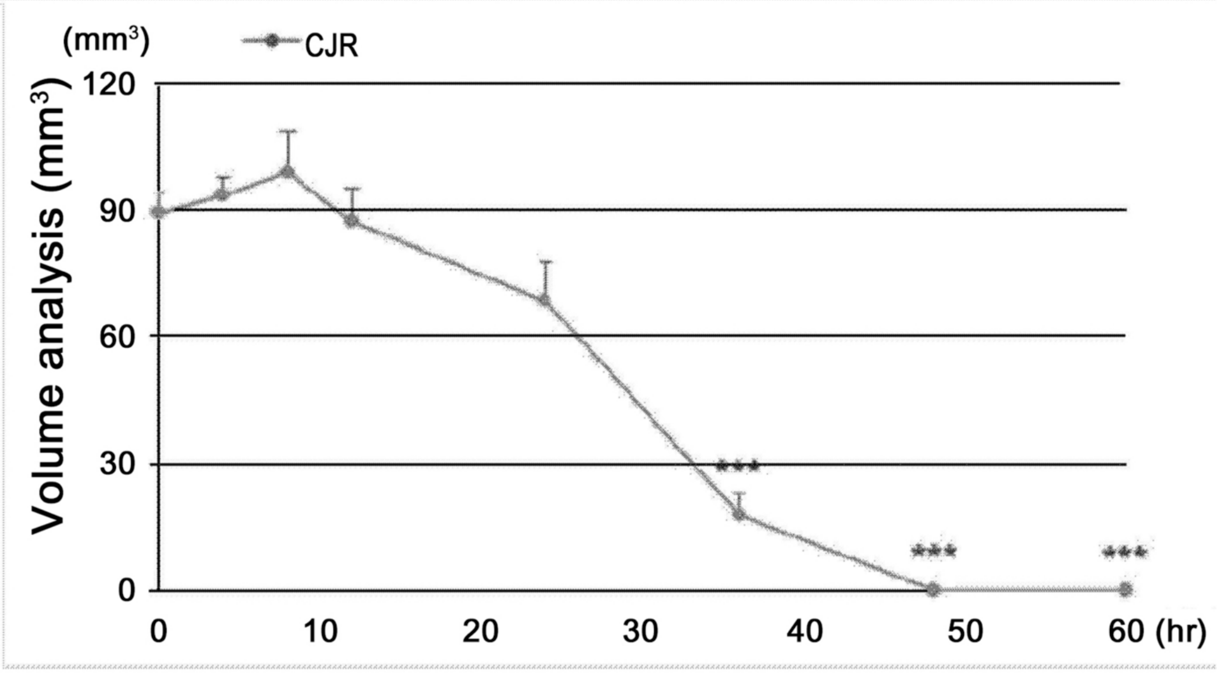
(mm$^3$)
CJR
Volume analysis (mm$^3$)
120
90
60
30
0
***
***
***
0
10
20
30
40
50
60 (hr)

[FIG. 6]

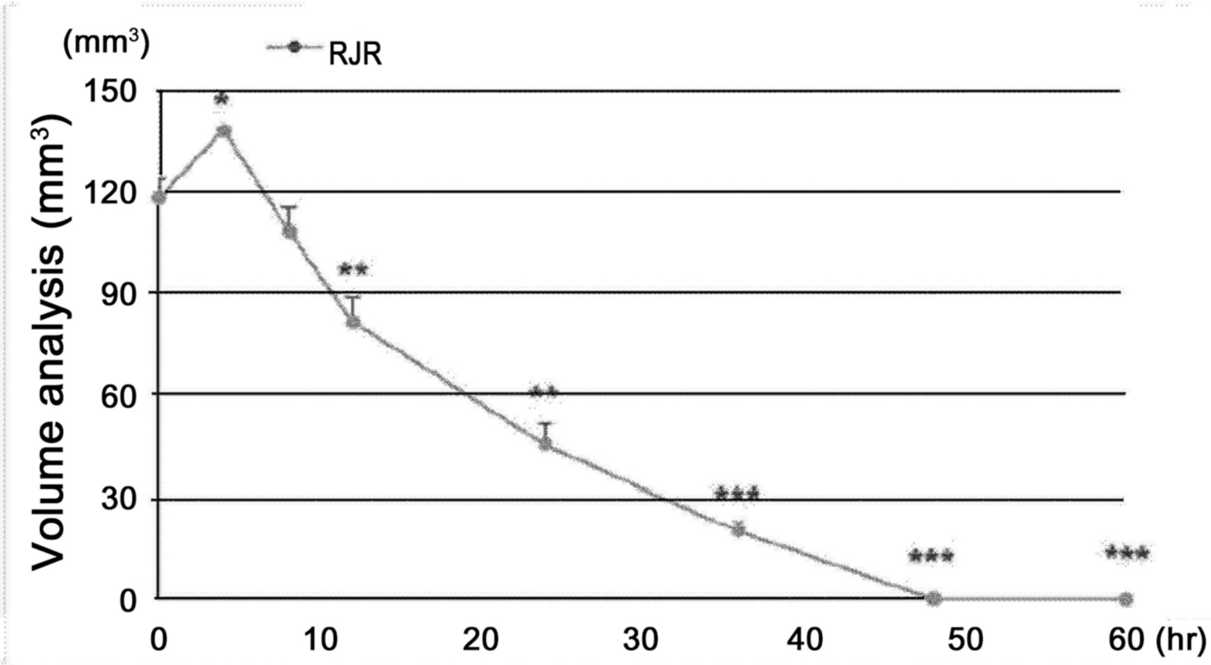
(mm$^3$)
RJR
Volume analysis (mm$^3$)
150
120
90
60
30
0
*
**
**
***
***
***
0
10
20
30
40
50
60 (hr)

[FIG. 7]

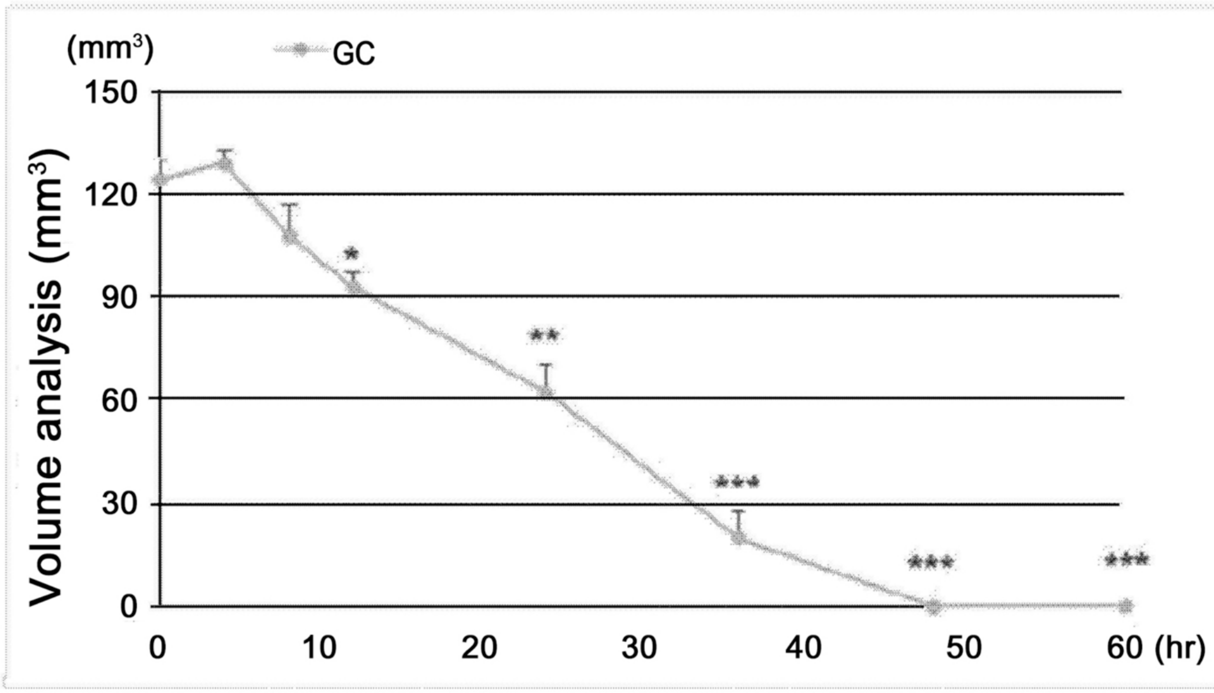
(mm3)
GC
Volume analysis (mm3)
150
120
90
60
30
0
*
**
***
***
***
0
10
20
30
40
50
60 (hr)

[FIG. 8]

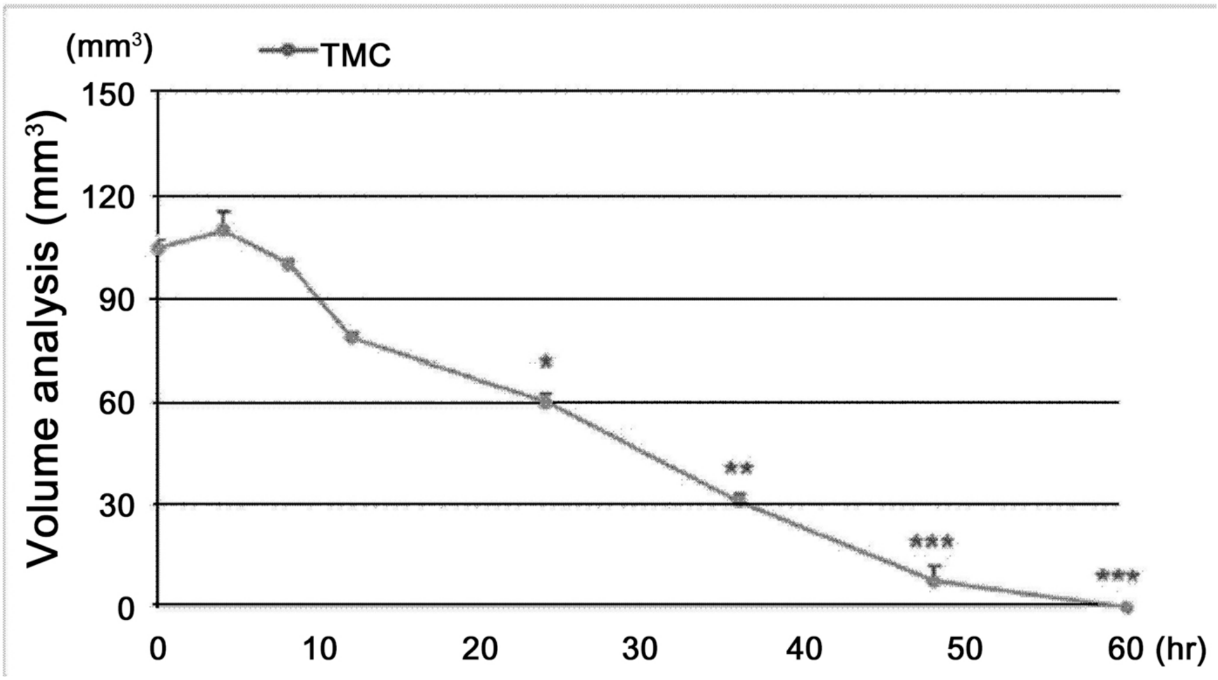
(mm3)
TMC
Volume analysis (mm3)
150
120
90
60
30
0
*
**
***
***
0
10
20
30
40
50
60 (hr)

[FIG. 9]

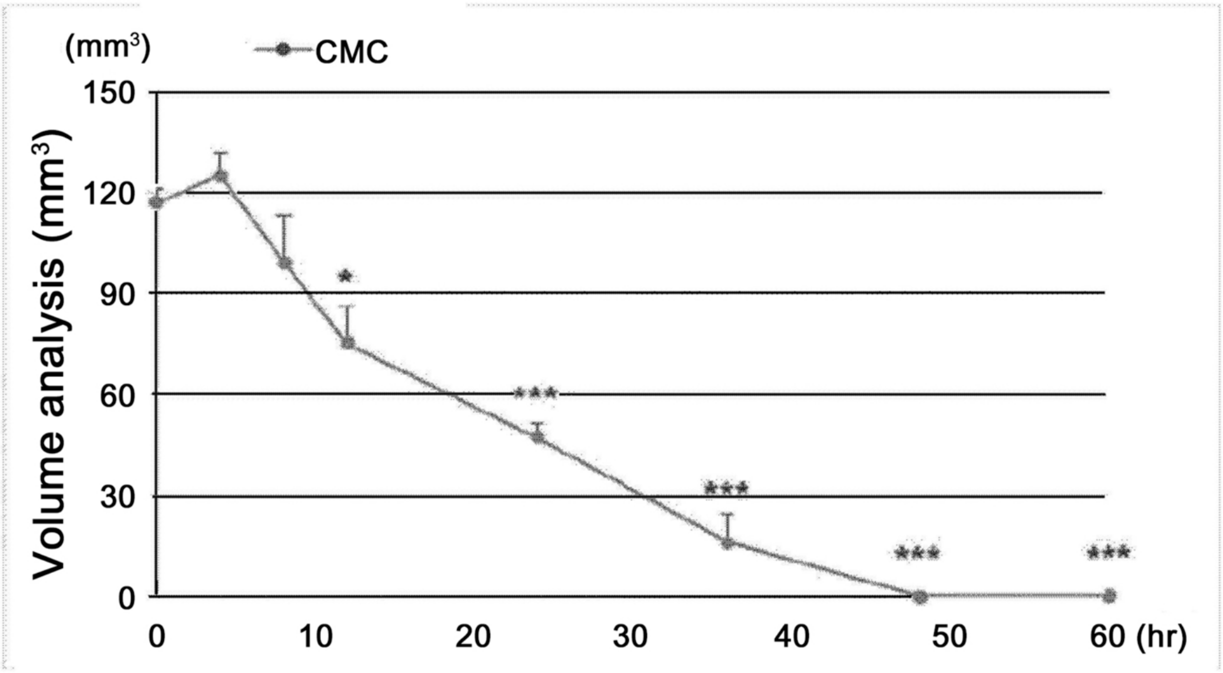
(mm3)
CMC
150
120
90
60
30
0
Volume analysis (mm3)
*
***
***
***
***
0
10
20
30
40
50
60 (hr)

[FIG. 10]

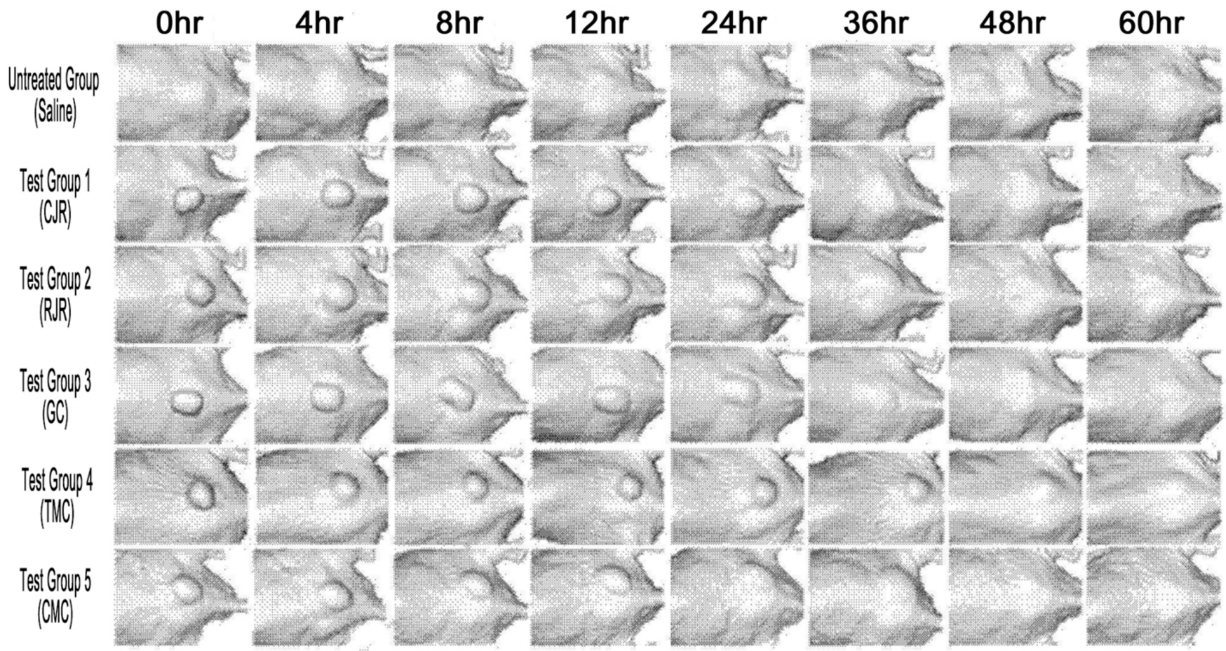
0hr
4hr
8hr
12hr
24hr
36hr
48hr
60hr
Untreated Group (Saline)
Test Group 1 (CJR)
Test Group 2 (RJR)
Test Group 3 (GC)
Test Group 4 (TMC)
Test Group 5 (CMC)

[FIG. 11]

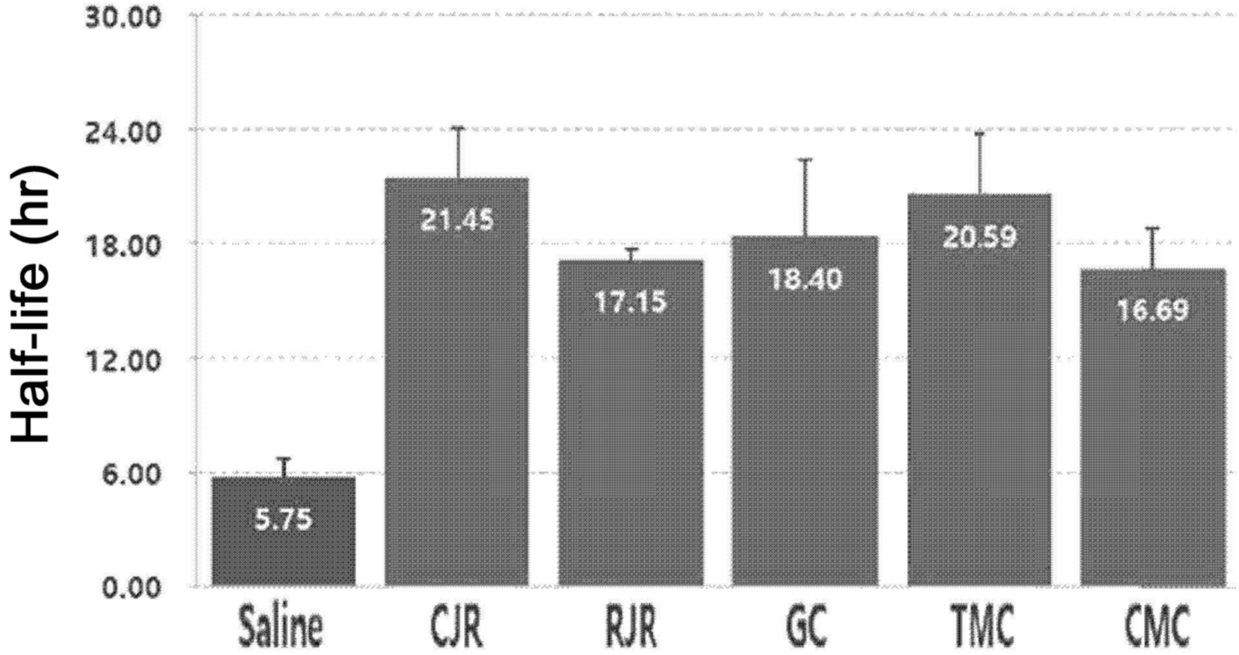
Half-life (hr)
30.00
24.00
18.00
12.00
6.00
0.00
5.75
21.45
17.15
18.40
20.59
16.69
Saline
CJR
RJR
GC
TMC
CMC

## INTERNATIONAL SEARCH REPORT

International application No. **PCT/KR2024/005349**

**A. CLASSIFICATION OF SUBJECT MATTER**

**A61L 27/20**(2006.01)i; **A61L 27/36**(2006.01)i; **A61L 27/26**(2006.01)i; **A61L 27/58**(2006.01)i

According to International Patent Classification (IPC) or to both national classification and IPC

**B. FIELDS SEARCHED**

Minimum documentation searched (classification system followed by classification symbols)

A61L 27/20(2006.01); A61K 31/7088(2006.01); A61K 31/722(2006.01); A61K 48/00(2006.01); A61K 9/51(2006.01); A61L 27/52(2006.01); A61L 27/54(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 핵산(nucleic acid), 글리콜 키토산(glycol chitosan), 트리메틸키토산(trimethyl chitosan), 카르복시메틸키토산(carboxymethyl chitosan), 필러(filler)

**C. DOCUMENTS CONSIDERED TO BE RELEVANT**

| Category* | Citation of document, with indication, where appropriate, of the relevant passages | Relevant to claim No. |
|---|---|---|
| Y | KR 10-1710639 B1 (PHARMARESEARCH PRODUCTS CO., LTD.) 08 March 2017 (2017-03-08) See paragraphs [0003], [0008]-[0009] and [0042]; claims 1, 5 and 9-10; and tables 1 and 3. | 1-15 |
| Y | US 2013-0196944 A1 (BARG, Heiko) 01 August 2013 (2013-08-01) See abstract; and paragraph [0063]. | 1-15 |
| A | KR 10-2019-0070343 A (ALLERGAN, INC.) 20 June 2019 (2019-06-20) See entire document. | 1-15 |
| A | KR 10-2010-0077155 A (ENGENE, INC.) 07 July 2010 (2010-07-07) See entire document. | 1-15 |
| A | EP 2745849 A1 (IDEA MEDICAL DEVICES S.R.L.) 25 June 2014 (2014-06-25) See entire document. | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 July 2024** | **19 July 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2024/005349**

| Patent document cited in search report | Publication date (day/month/year) | Patent family member(s) | Publication date (day/month/year) |
|---|---|---|---|
| KR 10-1710639 B1 | 08 March 2017 | CN 109195642 A | 11 January 2019 |
| | | CN 109195642 B | 10 December 2021 |
| | | WO 2017-213285 A1 | 14 December 2017 |
| US 2013-0196944 A1 | 01 August 2013 | EP 2605746 A2 | 26 June 2013 |
| | | US 2015-0335784 A1 | 26 November 2015 |
| | | WO 2012-022478 A2 | 23 February 2012 |
| | | WO 2012-022478 A3 | 13 December 2012 |
| KR 10-2019-0070343 A | 20 June 2019 | CN 109890428 A | 14 June 2019 |
| | | CN 109890428 B | 10 June 2022 |
| | | EP 3525834 A1 | 21 August 2019 |
| | | EP 3525834 B1 | 14 April 2021 |
| | | EP 3895742 A1 | 20 October 2021 |
| | | JP 2019-531816 A | 07 November 2019 |
| | | JP 6827108 B2 | 10 February 2021 |
| | | US 2020-0054786 A1 | 20 February 2020 |
| | | US 2023-0277725 A1 | 07 September 2023 |
| | | WO 2018-071033 A1 | 19 April 2018 |
| KR 10-2010-0077155 A | 07 July 2010 | CN 101873867 A | 27 October 2010 |
| | | CN 101873867 B | 23 September 2015 |
| | | EP 2195035 A1 | 16 June 2010 |
| | | EP 2195035 A4 | 22 February 2012 |
| | | EP 2195035 B1 | 06 December 2017 |
| | | JP 2010-540468 A | 24 December 2010 |
| | | JP 2014-208696 A | 06 November 2014 |
| | | JP 5650533 B2 | 07 January 2015 |
| | | KR 10-1761289 B1 | 04 August 2017 |
| | | KR 10-2015-0115964 A | 14 October 2015 |
| | | US 10647785 B2 | 12 May 2020 |
| | | US 2011-0039917 A1 | 17 February 2011 |
| | | US 2014-0350091 A1 | 27 November 2014 |
| | | US 2018-0215839 A1 | 02 August 2018 |
| | | US 8722646 B2 | 13 May 2014 |
| | | US 9850323 B2 | 26 December 2017 |
| | | WO 2009-039657 A1 | 02 April 2009 |
| EP 2745849 A1 | 25 June 2014 | EP 2745849 B1 | 27 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020140072008